(19) Europäisches Patentamt / European Patent Office / Office européen des brevets

(11) **EP 4 644 530 A1**

(12) **EUROPEAN PATENT APPLICATION**
published in accordance with Art. 153(4) EPC

(43) Date of publication:
**05.11.2025 Bulletin 2025/45**

(21) Application number: 23910813.7

(22) Date of filing: **28.12.2023**

(51) International Patent Classification (IPC):
*C12N 1/20* (2006.01)       *C12P 1/04* (2006.01)
*A61K 35/74* (2015.01)      *A61K 8/99* (2017.01)
*A61P 29/00* (2006.01)      *A61P 17/18* (2006.01)
*A61P 17/16* (2006.01)      *A61Q 19/02* (2006.01)
*A61Q 19/08* (2006.01)      *C12R 1/01* (2006.01)

(86) International application number:
**PCT/CN2023/142667**

(87) International publication number:
**WO 2024/140896 (04.07.2024 Gazette 2024/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.12.2022   CN 202211695333**

(71) Applicants:
• **Bloomage Biotechnology Corporation Limited**
  **Jinan, Shandong 250101 (CN)**
• **Bloomage Biotech (Tianjin) Co., Ltd.**
  **Tianjin 300270 (CN)**

(72) Inventors:
• **SUN, Shaojing**
  **Tianjin 300270 (CN)**
• **JIN, Yan**
  **Jinan, Shandong 250101 (CN)**
• **WEN, Liangliang**
  **Jinan, Shandong 250101 (CN)**

• **MA, Liang**
  **Tianjin 300270 (CN)**
• **LU, Zhen**
  **Jinan, Shandong 250101 (CN)**
• **WANG, Ruiyan**
  **Jinan, Shandong 250101 (CN)**
• **WANG, Enxu**
  **Tianjin 300270 (CN)**
• **ZHOU, Xianlong**
  **Tianjin 300270 (CN)**
• **GUO, Xueping**
  **Jinan, Shandong 250101 (CN)**

(74) Representative: **Abel & Imray LLP**
**Westpoint Building**
**James Street West**
**Bath BA1 2DA (GB)**

Remarks:
The complete document including Reference
Table(s) and the Sequence Listing(s) can be
downloaded from the EPO website

(54) **LEUCONOSTOC AND USE THEREOF**

(57)    The present application discloses leuconostoc HX-236 and a use thereof. HX-236 takes *Cordyceps militaris* powder as a unique nitrogen source, and a fermentation filtrate can be obtained by means of fermentation culture. The fermentation filtrate comprises high contents of cordycepin and cordycepic acid, and can improve the expression of hypoxia-inducible factors (HIFs) $1\alpha$ in cells, so that downstream signaling pathways for regulation of inflammatory response, cell autophagy, and tissue regeneration are activated, thereby alleviating the problems of skin aging such as cell viability decline, collagen loss, pigmentation, and skin elasticity reduction caused by hypoxia of the skin.

**Description**

**TECHNICAL FIELD**

The present application relates to a *Leuconostoc* sp. and use of the *Leuconostoc* sp. in fermenting *Cordyceps militaris,* and use of a fermentation product obtained by fermenting *Cordyceps militaris* with the *Leuconostoc* sp. in the fields of medicine and cosmetics.

**BACKGROUND ART**

The skin is the earliest tissue that shows the aging of the body. Skin aging has the following characteristics: increased wrinkles, relaxed skin, decreased luster, smoothness, tension, and elasticity, thicker skin texture, disordered skin grooves, increased pigmentation, and so on, which are all the enemies of beauty. For this, people usually use anti-aging skincare products to reduce the marks left on their face by time. Skin aging is mainly divided into natural aging and exogenous aging. Natural aging refers to the phenomenon in which the body experiences a decrease in cell vitality, cell migration and proliferation rate due to force majeure (such as gravity, changes in endocrine and immune function in the body), and genetic and other factors. During this process, the oxygen consumption of cells increases, causing the amount of oxygen in the skin to be insufficient to maintain cell vitality, which leads to the decline of cell vitality, the loss of collagen, pigmentation, skin laxity, wrinkles, and other signs. Exogenous aging refers to the phenomenon in which the prolonged exposure of the skin to environment (such as work pressure, staying up late, smoking and drinking, UV radiation, and exposure to chemicals) and other external factors ultimately leads to an increase in metabolic burden on the body, an increase in oxygen demand from other organs, a decrease in the amount of oxygen allocated to the skin, which is further manifested as the thickening and deepening of wrinkles, sagging of the skin, dilation of blood vessels, roughness of the epidermis, accumulation of degradation products, and other signs. With the improvement of people's consumption awareness and health concept, the selection of anti-aging products is also undergoing fundamental changes. However, most of the anti-aging cosmetics currently available on the market mainly alleviate skin aging by providing sufficient skin hydration, lacking full efficacy in improving cellular hypoxia and anti-aging issues.

*Cordyceps militaris* is a type species in the phylum *Ascomycota,* order *Hypocreales,* family *Clavicipitaceae,* and genus *Cordyceps,* also known as northern Cordyceps sinensis or northern Cordyceps, Cordyceps for short. The northern Cordyceps which is cultivated from living insect pupae is generally called Cordycep smilitaris. The main active ingredients in *Cordyceps militaris* include cordycepin and cordycepic acid, in which the cordycepic acid has the function of anti-oxidation and anti-free radical; and the cordycepin is the first nucleoside antibiotic isolated from fungi, which has unique antibacterial and antiviral effects and can significantly inhibit various inflammatory factors. Therefore, *Cordyceps militaris* is a promising raw material for skin care.

It has been reported that after fermentation with lactic acid bacteria, the content of active ingredients in *Cordyceps militaris* can be further increased. At the same time, the peptides and extracellular polysaccharides in the fermentation products of lactic acid bacteria have been proven to be natural antioxidants, and have significant effects in clearing free radicals and reducing oxidative damage. Enzymes and other effective factors in lactic acid bacteria extract can promote skin wound healing, and lactic acid bacteria lysate can improve skin hydration, enhance skin barrier, and resist photoaging. Therefore, as a new type of natural fermentation product, the lactic acid bacteria fermentation extract of *Cordyceps militaris* has broad application prospects in the pharmaceuticals, cosmetics and other fields.

**SUMMARY**

Aiming at the problems of skin inflammation, decreased cell vitality, collagen loss, pigmentation, etc. caused by skin hypoxia, resisting cell damage and aging, strengthening the physical barrier function of the skin, and maintaining the homeostasis of the skin's internal environment are good solutions. The present application provides a *Leuconostoc* sp., which has been verified to have a good effect on the release of effective ingredients in *Cordyceps militaris.* The *Cordyceps militaris* fermentation product obtained has the functions of regulating skin inflammation response, soothing, antioxidation, improving cell autophagy activity, promoting tissue regeneration, and promoting collagen endogeny, and has good application prospects in the fields of medicine, cosmetics, etc.

The specific technical solution of the present application is as follows:

A *Leuconostoc* sp. HX-236, wherein the *Leuconostoc* sp. HX-236 was screened from discarded bagasse, and has been deposited at the China Center for Type Culture Collection (CCTCC) on September 8, 2022, wherein the address is Wuhan University, Bayi Road, Wuchang District, Wuhan City, China, and the deposit number is CCTCC NO: M20221389。

The present application also provides a microbial agent comprising the above *Leuconostoc* sp. HX-236. The microbial agent can be a solid or liquid preparation.

The present application also provides use of the above *Leuconostoc* sp. HX-236 or the above microbial agent comprising the *Leuconostoc* sp. HX-236 in the preparation of a fermentation product. In the use, generally, the *Leuconostoc* sp. HX-236 or the above microbial agent are added to the substrate for fermentation to obtain the fermentation product. According to existing technology reports, the *Leuconostoc sp.* HX-236 in this application can replace other *Leuconostoc* sp. in existing technology for product fermentation. The specific fermentation method can refer to existing technology.

Preferably, the *Leuconostoc* sp. HX-236 or the above microbial agent comprising the *Leuconostoc* sp. HX-236 is used for fermenting *Cordyceps militaris* to obtain the *Cordyceps militaris* fermentation product.

The present application also provides a method for preparing a *Cordyceps militaris* fermentation product, which comprises the step of fermenting *Cordyceps militaris* with *Leuconostoc* sp. HX-236, and the product obtained by fermenting is the *Cordyceps militaris* fermentation product. In addition to the step of fermenting, the method can also comprises steps of performing subsequent process on the fermentation broth, including but not limited to pH adjustment, filtration, sterilization, protein removal, concentration, drying, and other operations.

Further, in the above method, the medium used for fermentation is composed of carbon source, nitrogen source, inorganic salt, water and other components, wherein glucose, fructose and other sugars can be selected as the carbon source, *Cordyceps militaris* powder can be selected as the nitrogen source, and sodium acetate, magnesium sulfate, etc. can be selected as the inorganic salt.

Preferably, in the above method, *Cordyceps militaris* is selected as the sole nitrogen source for the fermentation medium. Those skilled in the art can understand that selecting *Cordyceps militaris* as the sole nitrogen source for fermentation medium means that no other nitrogen source can be added.

In a specific embodiment of the present application, the formula for the fermentation medium adopts the medium formula disclosed in CN111808901A.

Further, in the above methods, the optimal temperature for culturing *Leuconostoc* sp. HX-236 is 35-38 °C.

Further, in the above method, the fermentation process comprises: adding the seed liquid of *Leuconostoc* sp. HX-236 into the fermentation medium, culturing under aeration and stirring at 35-38°C, without controlling the pH value during the fermentation process, and ending the fermentation when there is no residual sugar to obtain the fermentation broth. The seed liquid of *Leuconostoc* sp. HX-236 can be obtained by culturing *Leuconostoc* sp. HX-236 in liquid MRS medium at a temperature of 35-38°C and a pH value of 6.2-6.6.

The present application also provides a *Cordyceps militaris* fermentation product, which is obtained by fermenting *Cordyceps militaris* with *Leuconostoc* sp. HX-236, preferably prepared using the method for preparing the *Cordyceps militaris* fermentation product described above. The fermentation product can be in either liquid or solid form. It can be fermentation broth obtained by fermenting *Cordyceps militaris* with *Leuconostoc* sp. HX-236, or a liquid or solid product obtained by performing further subsequent process on the fermentation broth. The subsequent processing includes but is not limited to adjusting the pH of the fermentation broth, filtering, sterilizing, protein removal, concentration, drying, and other operations.

Preferably, in *Cordyceps militaris* fermentation product, the content of cordycep is 80-200mg/L, for example, it may be 80mg/L, 90mg/L, 100mg/L, 110mg/L, 120mg/L, 130mg/L, 140mg/L, 150mg/L, 160mg/L, 170mg/L, 180mg/L, 190mg/L, 200mg/L, etc; the content of cordycepic acid is 5-30g/L, for example, it may be 5g/L, 6g/L, 7g/L, 8g/L, 9g/L, 10g/L, 15g/L, 20g/L, 25g/L, 30g/L, etc.

Further, the fermentation product is rich in various natural active ingredients, has multiple functions, and has good application prospects in the pharmaceuticals, cosmetics and other fields.

The present application also provides use of above *Leuconostoc* sp. HX-236, the above microbial agent, or the above *Cordyceps militaris* fermentation product in the preparation of medicine and cosmetics.

The present application also provides a composition comprising the above *Leuconostoc* sp. HX-236, or the above bacterial agent, or the above *Cordyceps militaris* fermentation product.

The present application also provides use of above *Leuconostoc* sp. HX-236, the above microbial agent, the above *Cordyceps militaris* fermentation product, or the above composition in anti-aging, promoting production of collagen I, promoting cell repair and regeneration, anti-inflammatory, whitening, or increasing skin elasticity.

The present application also provides a method for anti-aging, promoting the production of collagen I, promoting cell repair and regeneration, anti-inflammatory, whitening, or increasing skin elasticity, which comprises using the above *Leuconostoc* sp. HX-236, the above microbial agent, the above *Cordyceps militaris* fermentation product, or the above composition.

The present application has independently screened a *Leuconostoc* sp. HX-236, which can ferment *Cordyceps militaris* and fully release the active ingredients in *Cordyceps militaris.* During the fermentation process, *Cordyceps militaris* is the sole nitrogen source. The resulting fermentation broth is rich in various natural active ingredients, and has high content of cordycepin acid and cordycepin, which is easily soluble in water, easy to use, and has good stability and compatibility, and thus can be widely used in various skincare products.

After verification, the *Cordyceps militaris* fermentation product obtained in this application can increase the expression level of hypoxia inducible factor HIF-1$\alpha$ (which can increase the expression level of skin hypoxia inducible factor HIF-1α by 2%-8%), thereby activating downstream signaling pathways that regulate inflammation response, cell autophagy, and tissue regeneration, restoring cell vitality, and also increasing the expression level of skin Collagen I and autophagy marker LC3B. Further, by regulating cell hypoxia pressure, it can improve skin aging problems caused by hypoxia, such as decreased cell vitality, collagen loss, pigmentation, and reduced skin elasticity, playing the functions of regulating skin inflammation response, promoting cell autophagy activity, promoting tissue regeneration, soothing and antioxidation, and promoting collagen endogeny. The *Cordyceps militaris* fermentation product can be used in drugs, cosmetics, and other fields.

**Deposit Information**

The *Leuconostoc* sp. HX-236 in the present application has been deposited at the China Center for Type Culture Collection (CCTCC) on September 8, 2022, wherein the deposit address is China Center for Type Culture Collection in the Wuhan University on Bayi Road, Wuchang District, Wuhan City, Hubei Province, China, with postal code 430072 and the deposit number is CCTCC NO: M20221389。

**BRIEF DESCRIPTION OF THE DRAWINGS**

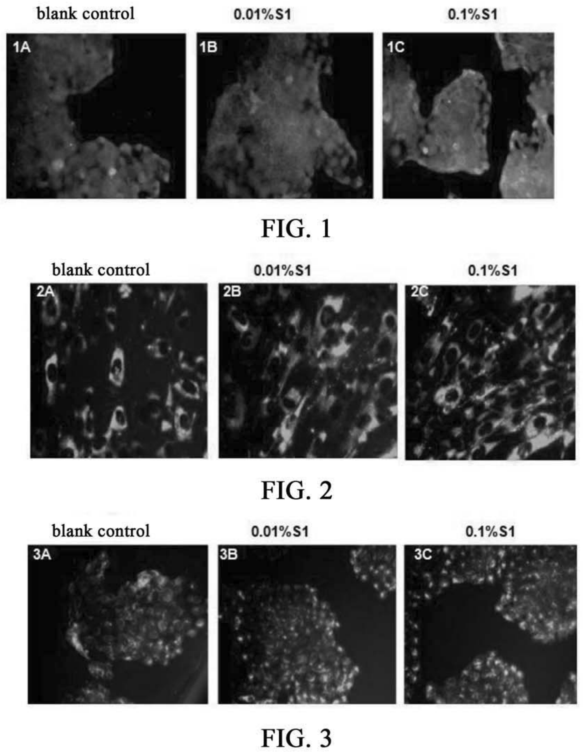

FIG. 1 shows the microscope image of the anti-aging efficacy test in Test Example 3. FIG. 1A shows the fluorescence microscope image of hypoxia inducible factor HIF-1α in the human immortalized epidermal cell (HaCaT) model without the addition of *Cordyceps militaris* fermentation filtrate. FIG. 1B shows the fluorescence microscope image of hypoxia inducible factor HIF-1α in the human immortalized epidermal cell (HaCaT) model with a concentration of 0.01% *Cordyceps militaris* fermentation filtrate. FIG. 1C shows the fluorescence microscope image of hypoxia inducible factor HIF-1α in the human immortalized epidermal cell (HaCaT) model with a concentration of 0.1% *Cordyceps militaris* fermentation filtrate.
FIG. 2 shows the microscope images of promoting collagen endogeny and damage repair test in Test Example 4. FIG. 2A shows the fluorescence microscope image of collagen I in the human primary dermal fibroblast (FB) model without the addition of *Cordyceps militaris* fermentation filtrate. FIG. 2B shows the fluorescence microscope image of collagen I in the human primary dermal fibroblast (FB) model with a concentration of 0.01% *Cordyceps militaris* fermentation filtrate. FIG. 2C shows the fluorescence microscope image of collagen I in the human primary dermal fibroblast (FB) model with a concentration of 0.1% *Cordyceps militaris* fermentation filtrate.
FIG. 3 shows the microscopy images of improving cell autophagy activity test in Test Example 5. FIG. 3A shows the fluorescence microscopy image of autophagy marker LC3B in the human immortalized epidermal cell (HaCaT) model without the addition of *Cordyceps militaris.* FIG. 3B shows the fluorescence microscopy image of autophagy marker LC3B in the human immortalized epidermal cell (HaCaT) model with a concentration of 0.01% *Cordyceps militaris* fermentation filtrate. FIG. 3C shows the fluorescence microscopy image of autophagy marker LC3B in the human immortalized epidermal cell (HaCaT) model with a concentration of 0.1% *Cordyceps militaris* fermentation filtrate.

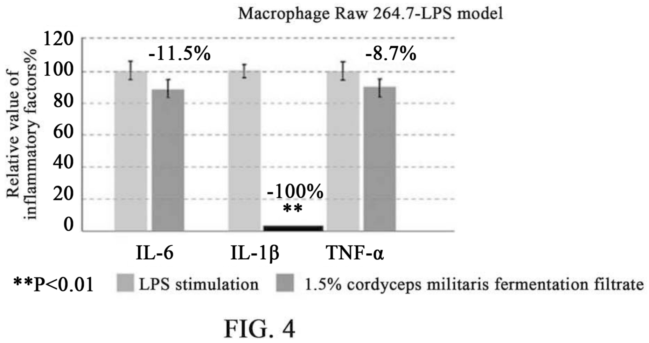

FIG. 4 shows the inhibition rate of inflammatory factors in soothing and anti-inflammatory test in Test Example 6.

**DETAILED DESCRIPTION**

In order to make the purpose, technical solution, and advantages of the present application clearer and more understandable, the following will provide further detailed explanations of the present application in conjunction with the accompanying drawings and examples. It should be understood that the specific examples described herein are only used to explain the present application and are not intended to limit the present application.
Unless otherwise specified, the following concentrations are mass percentage concentrations.

**Example** 1

Screening and Identification of *Leuconostoc* sp. HX-236

The bagasse as raw material was diluted gradiently and dispersed with sterile water. Each solution was evenly coated on an MRS solid culture medium plate using a coating machine, and incubated in the incubator at 30°C for 1-2 days. Single colonies with smooth and rounded colony morphology were selected and inoculated onto MRS solid culture medium containing calcium carbonate, and were incubated in the incubator at 30°C for 1-2 days. The single colonies that produced calcium dissolving zone were selected and repeatedly purified by streaking. Pure strains were stored on the inclined surface and in a refrigerator at 4°C. The composition of MRS solid culture medium is: 10g/L peptone, 5g/L beef extract, 5g/L yeast powder, 2g/L dipotassium hydrogen phosphate, 2g/L diammonium citrate, 5g/L sodium acetate, 20g/L glucose, 1mL/L Tween 80, 2g/L magnesium sulfate, 2g/L manganese sulfate, and 20g/L agar powder. The composition of MRS solid culture medium containing calcium carbonate is: 10g/L peptone, 5g/L beef extract, 5g/L yeast powder, 2g/L dipotassium hydrogen phosphate, 2g/L diammonium citrate, 5g/L sodium acetate, 20g/L glucose, 1mL/L Tween 80, 2g/L magnesium sulfate, 2g/L manganese sulfate, 20g/L agar powder and 5g/L calcium carbonate.

The above deposited strains were used to fermentate *Cordyceps militaris,* and the strain with the highest content of cordycepin and cordycepic acid in the fermentation broth was taken as the target strain. The fermentation method was as follows: culturing under aeration and stirring at 35-38°C, with a rotation speed of 200 r/min and an aeration rate of 0.25 vvm. The pH value was not controlled during the fermentation process, and the fermentation was ended when there was no residual sugar, to obtain the fermentation broth.

The selected target strain was named HX-236 and sent to Sangon Biotech (Shanghai) Co., Ltd. for genome sequencing identification. The results show that the strain is *Leuconostoc* sp. The sequencing result of the 16sRNA gene of this strain is shown in SEQ ID NO: 1:

CTCAGGATGAACGCTGGCGGCGTGCCTAATACATGCAAGTCGAACGCACAGCGAAAGGTGCTTGCACCTTTCAAGTGA
GTGGCGAACGGGTGAGTAACACGTGGACAACCTGCCTCAAGGCTGGGGATAACATTTGGAAACAGATGCTAATACCGA
ATAAAACTTAGTGTCGCATGACACAAAGTTAAAAGGCGCTTCGGCGTCACCTAGAGATGGATCCGCGGTGCATTAGTTA
GTTGGTGGGGTAAAGGCCTACCAAGACAATGATGCATAGCCGAGTTGAGAGACTGATCGGCCACATTGGGACTGAGAC
ACGGCCCAAACTCCTACGGGAGGCTGCAGTAGGGAATCTTCCACAATGGGCGAAAGCCTGATGGAGCAACGCCGCGTG
TGTGATGAAGGCTTTCGGGTCGTAAAGCACTGTTGTATGGGAAGAACAGCTAGAATAGGAAATGATTTTAGTTTGACGG
TACCATACCAGAAAGGGACGGCTAAATACGTGCCAGCAGCCGCGGTAATACGTATGTCCCGAGCGTTATCCGGATTTATT

GGGCGTAAAGCGAGCGCAGACGGTTTATTAAGTCTGATGTGAAAGCCCGGAGCTCAACTCCGGAATGGCATTGGAAAC
TGGTTAACTTGAGTGCAGTAGAGGTAAGTGGAACTCCATGTGTAGCGGTGGAATGCGTAGATATATGGAAGAACACCAG
TGGCGAAGGCGGCTTACTGGACTGCAACTGACGTTGAGGCTCGAAAGTGTGGGTAGCAAACAGGATTAGATACCCTGG
TAGTCCACACCGTAAACGATGAACACTAGGTGTTAGGAGGTTTCCGCCTCTTAGTGCCGAAGCTAACGCATTAAGTGTT
CCGCCTGGGGAGTACGACCGCAAGGTTGAAACTCAAAGGAATTGACGGGGACCCGCACAAGCGGTGGAGCATGTGGT
TTAATTCGAAGCAACGCGAAGAACCTTACCAGGTCTTGACATCCTTTGAAGCTTTTAGAGATAGAAGTGTTCTCTTCGG
AGACAAAGTGACAGGTGGTGCATGGTCGTCGTCAGCTCGTGTCGTGAGATGTTGGGTTAAGTCCCGCAACGAGCGCAA
CCCTTATTGTTAGTTGCCAGCATTCAGATGGGCACTCTAGCGAGACTGCCGGTGACAAACCGGAGGAAGGCGGGGACG
ACGTCAGATCATCATGCCCCTTATGACCTGGGCTACACACGTGCTACAATGGCGTATACAACGAGTTGCCAACCCGCGA
GGGTGAGCTAATCTCTTAAAGTACGTCTCAGTTCGGATTGTAGTCTGCAACTCGACTACATGAAGTCGGAATCGCTAGTA
ATCGCGGATCAGCACGCCGCGGTGAATACGTTCCCGGGTCTTGTACACACCGCCCGTCACACCATGGGAGTTTGTAATG
CCCAAAGCCGGTGGCCTAACCTTTTAGGAAGGAGCCGTCTAAGGCAGGACAGATGACTGGGGTGAAGTCGT

The morphological characteristics of the *Leuconostoc* sp. HX-236 are as follows: the *Leuconostoc* sp. has circular or bean shaped colony morphology, and the colony has a diameter of less than 1.0 mm, smooth surface, and milky white color, and the *Leuconostoc* sp.does not produce any pigments; the cell morphology is spherical, bean shaped, or short stem shaped, with some arranged in pairs or short chains, immobile, and without spores; and it is positive in gram staining.

## Example 2

Preparation of seed liquid: the *Leuconostoc* sp. HX-236 was inoculated into liquid MRS medium, with pH value of 6.2-6.6, and incubated in a shaking flask at 37°C for 2 days to obtain seed liquid; the liquid MRS medium consists of 10g/L peptone, 5g/L beef extract, 5g/L yeast powder, 2g/L dipotassium hydrogen phosphate, 2g/L diammonium citrate, 5g/L sodium acetate, 20g/L glucose, 1mL/L Tween 80, 2g/L magnesium sulfate, and 2g/L manganese sulfate.

Fermentation culture: the seed liquid was inoculated into the fermentation medium at an amount of 5wt%. The formula of the fermentation medium is: 8g/L *Cordyceps militaris* powder, 5g/L glucose, 18g/L fructose, 5g/L sodium acetate, 2g/L magnesium sulfate, balance being water, pH value of 6.2-6.6. The culture was performed under aeration and stirring at 37 °C, with a speed of 200 r/min and an aeration rate of 0.25 vvm. The pH value was not controlled during the fermentation process, and the fermentation was terminated when there was no residual sugar, obtaining the fermentation broth. The fermentation broth was heated in a water bath of 65°C for 1h, and then was adjusted to the pH value of 5.0~6.0, and passed through a 0.45 μm membrane to obtain *Cordyceps militaris* fermentation filtrate S1.

**Comparative Example 1**

The *Cordyceps militaris* fermentation filtrate D1 was prepared by using the method described in Example 2, except that the *Leuconostoc* sp. HX-236 was replaced with *Leuconostoc mesenteroides subsp. dextranicum* SHBCC D14110.

**Comparative Example 2**

The *Cordyceps militaris* fermentation filtrate D2 was prepared by using the method described in Example 2, except that the *Leuconostoc* sp. HX-236 was replaced with *Leuconostoc mesenteroides subsp. dextranicum* SHBCC D16368.

**Comparative Example 3**

The fermentation medium of Example 2 was not inoculated with strain, and was heated in a water bath of 65°C for 1h, and then was adjusted to the pH value of 5.0~6.0, and passed through a 0.45 μm membrane to obtain *Cordyceps militaris* extract D3.

Test Example 1: **Cordycepin Content**

The specific steps for determining the cordycepin content in the sample prepared in each of Example and Comparative Example were as follows:

1.1 Preparation of a sample solution: the sample of each of Example and Comparative Example was diluted by 10 times to obtain the sample solution.

1.2 Preparation of a standard sample solution: 1 mg of cordycepin as a standard sample was accurately weighed, added with 50 mL of distilled water for dissolution, and then was transferred to a 100 mL volumetric flask, and added with water to the scale. The solution was diluted 10 times prior to use to obtain the standard sample solution.

1.3 Chromatographic conditions: detection was performed on a liquid chromatograph, in which the column is: CAPCELL PAK-C18 column, and the mobile phase was an aqueous solution containing 0.04M potassium dihydrogen phosphate and 5wt% acetonitrile, with a flow rate of 1.0 ml/min and a column temperature of 35°C, the detection wavelength was 260 nm and the sample volume was 10 μl.

1.4 Determination: the cordycepin content was calculated based on the peak area, and the results are shown in Table 1 below.

Table 1 Cordycepin Content

|  | S1 | D1 | D2 | D3 |
|---|---|---|---|---|
| Cordycepin Content (ppm) | 182.6 | 117.4 | 119.3 | 108.1 |

**Test Example 2: Cordycepic acid Content**

2.1. Reagent preparation:

Nash reagent: it requires fresh preparation. 150 g of ammonium acetate was accurately weighed and dissolved with distilled water. 2 mL of glacial acetic acid and 2 mL of acetylacetone were added. The volume of the solution was made up to a 1000 ml.

0.015mol/L sodium periodate solution: 3.2 g of sodium periodate was accurately weighed and dissolved in 0.12 mol/L hydrochloric acid.

0.1% L-rhamnose solution: 0.1g of L-rhamnose was accurately weighed and dissolved in distilled water, and the volume of the solution was made up to a 100 ml.

Preparation (reserve) of cordycepic acid standard solution: 100 mg of cordycepic acid standard was weighted and dissolved in distilled water, and the volume of the solution was made up to a 100 ml to obtain the 1mg/ml cordycepic acid standard solution. The cordycepic acid standard solution was diluted ten times prior to use.

Test solution: samples prepared in each of Examples and Comparative Examples were diluted 400 times.

2.2. Determination method:

(1) Preparation of standard curve for cordycepic acid

Table 2 Sample system for the standard curve of cordycepic acid

| Cordycepic acid content (μg/ml) | 0 | 20 | 40 | 60 | 80 | 100 |
|---|---|---|---|---|---|---|
| Cordycepic acid standard solution (ml) | 0 | 0.2 | 0.4 | 0.6 | 0.8 | 1 |
| Water (ml) | 1 | 0.8 | 0.6 | 0.4 | 0.2 | 0 |
| Sodium periodate solution (ml) | 1 | 1 | 1 | 1 | 1 | 1 |
| Rhamnose solution (ml) | 2 | 2 | 2 | 2 | 2 | 2 |
| Nash agent (ml) | 4 | 4 | 4 | 4 | 4 | 4 |

The cordycepic acid standard solution was mixed with water according to the above Table 2, and then 1ml of sodium periodate solution was added respectively and mixed. The solution was leaved at room temperature for 10 minutes.

2ml of 0.1% rhamnose solution was added in each test tube to remove excess sodium periodate. After shaking and mixing, 4ml of freshly prepared Nash reagent was added. The solution was kept in a 53°C water bath for 15 minutes, and then quickly cooled to room temperature. The absorbance value was measured at 412 nm and the standard curve of Cordycepic acid content (μ g/ml) vs. A412 was plotted.

(2) Sample test

50 μl of each test solution was taken and diluted into a 100ml volumetric flask. Then 1ml of the resulting solution was taken and placed in a stoppered test tube. For the blank control, 1ml of water was added, and then 1ml of sodium periodate solution was added, mixed well, and let it stand at room temperature for 10 minutes. 2ml of 0.1% rhamnose was added to each test tube to remove excess sodium periodate, and shaken well. 4ml of freshly prepared Nash reagent was added, incubated in a 53°C water bath for 15 minutes, and then quickly cooled to room temperature. The absorbance value was measured at 412 nm. The absorbance value was input into the standard curve, and then multiplied by the dilution factor to obtain the oxalic acid content. The results are shown in Table 3.

Table 3 Cordycepic acid content

| | S1 | D1 | D2 | D3 |
|---|---|---|---|---|
| Cordycepic acid content (g/L) | 16.83 | 2.9 | 3.7 | 0.61 |

**Test Example 3: Anti-aging effect test**

Establishment experiment of human immortalized epidermal cell model

1. Group setting

Control group: DMEM medium containing 10% FBS.
Experimental group: DMEM medium containing 10% FBS, in which 0.01%, or 0.1% *Cordyceps militaris* fermentation filtrate S1 is contained.

2. Experimental steps

(1) Cell inoculation: HaCaT cells were taken and cultured in DMEM medium in a T75 culture bottle until the cell density was about 80%. Then the HaCaT cells were inoculated onto a 24-well plate with a cover glass and cultured at 37°C under 5% $CO_2$.

(2) Sample processing: after 24 hours, the supernatant was suctioned off. The samples added in the experimental group were complete medium containing 0.01%, or 0.1% *Cordyceps militaris* fermentation filtrate S1 while a control group was set. The cells were incubated for 72 hours in an incubator at 37°C under 5% $CO_2$.

(3) Immunofluorescence detection: the supernatant was sucked off, and the cells were washed twice and fixed with ice methanol at -20°C, and then were added with HIF-1$\alpha$ primary antibody respectively and incubated overnight at 4°C. The cells were washed three times with PBS the next day, added with the corresponding secondary antibody, and incubated at room temperature in the dark for 1.5 hours. The nuclear staining was performed with DAPI, and the slice was mounted, and then observation was performed under a light microscope and photos were taken.

Immunofluorescence method is based on the principle of antigen antibody reaction. First, a known antigen or antibody is labeled with fluorescein to make a fluorescent label, and then this fluorescent antibody (or antigen) is used as a molecular probe to examine the corresponding antigen (or antibody) in cells or tissues. The antigen antibody complex formed in cells or tissues contains fluorescein. By observing the specimen under a fluorescence microscope, the cells or tissues where the fluorescence is located can be seen as fluorescein emits bright fluorescence (yellow green or orange red) under the irradiation of excited light, thereby determining the nature and location of the antigen or antibody, and using quantitative techniques to determine the content.

2. Detection of HIF-1$\alpha$ content

Image J software was used to semi quantitatively analyze the fluorescence data of the photos. After removing the background, the reading was homogenized using the control as a reference and represented as Means $\pm$ SEM. The Graphpad Prism statistical software was used for analysis and processing. The results are shown in Table 4 below.

Table 4 HIF-1$\alpha$ content in human immortalized epidermal cell HaCaT

|  | Control group | Experimental group 0.01% *Cordyceps militaris* fermentation filtrate S1 | Experimental group 0.1% *Cordyceps militaris* fermentation filtrate S1 |
|---|---|---|---|
| HIF-1$\alpha$ content (%) | 100% | 102% | 108%* |
| Note: * indicates p<0.05 | | | |

The microscope images of the detection results are shown in FIGS. 1A-1C. The green fluorescence sites are displayed as a gray area in the black and white images. The denser the distribution, the higher the brightness, and the higher the expression level of HIF-1$\alpha$. As can be seen from FIGS. 1A-1C, compared with the control group, the expression levels of HIF-1$\alpha$ are increased by adding Cordyceps pupis fermentation filtrates, wherein when 0.1% *Cordyceps militaris* fermentation filtrate is added, the expression level of HIF-1$\alpha$ has the most significant increase, and the expression level of HIF-1 $\alpha$ factor is significantly upregulated by 8%, thereby improving skin hypoxia and enhancing cell vitality.

**Test Example 4: Test of promotion of collagen endogeny and damage repair**

1. Immunohistochemical detection of collagen I in human primary dermal fibroblasts (FB)

1. Group setting

Control group: DMEM medium containing 10% FBS.

Experimental group: DMEM medium containing 10% FBS, in which 0.01%, or 0.1% *Cordyceps militaris* fermentation filtrate S1 is contained.

2. Experimental steps

(1) Cell inoculation: FB cells were taken and cultured in DMEM medium in a T75 culture bottle until the cell density was about 80%. Then the FB cells were inoculated onto a 24-well plate with a cover glass and cultured at 37°C under 5% $CO_2$.

(2) Sample processing: after 24 hours, the supernatant was suctioned off. The complete medium containing 0.01%, or 0.1% *Cordyceps militaris* fermentation filtrate S1 was added in the experimental group, while a control group was set.

The cells were incubated for 72 hours in an incubator at 37°C under 5% $CO_2$.

(3) Immunofluorescence detection: the supernatant was sucked off, and the cells were washed twice and fixed with ice methanol at -20°C, and then were added Collagen I primary antibody respectively and incubated overnight at 4°C. The cells were washed three times with PBS the next day, added with the corresponding secondary antibody, and incubated at room temperature in the dark for 1.5 hours. The nuclear staining was performed with DAPI, and the slice was mounted, and then observation was performed under a light microscope and photos were taken. Finally, Image J software was used to semi quantitatively analyze the fluorescence data of the photos.

2. Quantitative analysis of collagen I in human primary dermal fibroblasts by immune assay

Image J software was used to semi quantitatively analyze the fluorescence data of the photos. After removing the background, the reading was homogenized using the control as a reference and represented as Means ± SEM. The Graphpad Prism statistical software was used for analysis and processing. The results are shown in Table 5 below.

Table 5 Content of collagen I in human primary dermal fibroblast FB

|  | Control group | Experimental group 0.01% | Experimental group 0.1% |
|---|---|---|---|
| Content of collagen I | 1.00 | 1.17 | 1.23 |
| Increased percentage | -- | 17% | 23%* |
| Note: * indicates p<0.05 | | | |

The microscope images of the detection results are shown in FIGS. 2A-2C. The denser the distribution of the green fluorescence sites (displayed as a gray area in the black and white images), the higher the brightness, and the higher the expression level of collagen I. As can be seen from FIGS. 2A-2C, compared with the control group, the expression levels of collagen I are increased by adding different concentrations of Cordyceps pupis fermentation filtrate, wherein the model with addition of 0.1% *Cordyceps militaris* fermentation filtrate has the most significant increase in the expression level of collagen I, indicating that the *Cordyceps militaris* fermentation filtrate can significantly upregulate the synthesis of collagen I. Compared with the control group, 0.01% and 0.1% *Cordyceps militaris* fermentation filtrate upregulate the expression level of collagen I by 17% and 23%, respectively, thereby making the skin elastic, moist, and plump.

**Test Example 5: Improvement of autophagy activity**

1. Group setting

Control group: DMEM medium containing 10% FBS.

Experimental group: DMEM medium containing 10% FBS, in which 0.01%, or 0.1% *Cordyceps militaris* fermentation filtrate S1 is contained.

2. Experimental steps

(1) Cell inoculation: HaCaT cells were taken and cultured in DMEM medium in a T75 culture bottle until the cell density was about 80%. Then the FB cells were inoculated onto a 24-well plate with a cover glass and cultured at 37°C under 5% $CO_2$.
(2) Sample processing: after 24 hours, the supernatant was suctioned off. The complete medium containing 0.01%, or 0.1% *Cordyceps militaris* fermentation filtrate S1 was added in the experimental group, while a control group was set. The cells were incubated for 72 hours in an incubator at 37°C under 5% $CO_2$.
(3) Immunofluorescence detection: the supernatant was sucked off, and the cells were washed twice and fixed with ice methanol at -20°C, and then were added with LC3B primary antibody respectively and incubated overnight at 4°C. The cells were washed three times with PBS the next day, added with the corresponding secondary antibody, and incubated at room temperature in the dark for 1.5 hours. The nuclear staining was performed with DAPI, and the slice was mounted, and then observation was performed under a light microscope and photos were taken.. Finally, Image J software was used to semi quantitatively analyze the fluorescence data of the photos. The results are shown in Table 6 below.

Table 6 Content of LC3B in human immortalized epidermal cell (HaCaT)

|  | Control group | Experimental group 0.01% | Experimental group 0.1% |
|---|---|---|---|
| Content of LC3B (%) | 1.00 | 1.12 | 1.15 |
| Increased percentage | - | 12% | 15%* |
| Note: ** indicates p<0.001 | | | |

The microscope images of the immunohistochemical detection results of autophagy marker LC3B are shown in FIGS. 3A-3C. The denser the distribution of green fluorescent sites, the higher the brightness, and the higher the expression level of autophagy marker LC3B. As can be seen from FIGS. 3A-3C, compared with the control group, the expression levels of autophagy marker LC3B are significantly increased by adding Cordyceps pupis fermentation filtrates, wherein the expression level of LC3B in cells is upregulated by 12% by adding 0.01% *Cordyceps militaris* fermentation filtrate, indicating that *Cordyceps militaris* fermentation filtrate can significantly improve the autophagy ability of cells, promote cell repair and regeneration, and thus help the skin resist damage and aging.

Test Example 6: Soothing and anti-inflammatory Test

The *Cordyceps militaris* fermentation filtrate S1 was used and diluted with water into a concentration of 1.5% (v/v) as the test sample, and the inhibitory effects of the test sample on three pro-inflammatory factors were determined at this concentration. The method was as follows:

Using a mouse macrophage model, LPS was used to stimulate the production of inflammatory factors and investigate whether the sample could inhibit the secretion of inflammatory factors. a mother solution of LPS with a concentration of 500000 units/mL was prepared with serum-free 1640 culture solution, filtered through a 0.22 $\mu$m membrane for sterilization, stored in a refrigerator at - 20°C, and diluted to 10000 units/mL of the action solution prior to use. The samples in contact with cells were prepared using the action solution of LPS.

Raw264.7 cells in logarithmic growth phase were taken, inoculated into a 24-well plate at $1 \times 10^5$/mL and cultured for 24 hours at 37°C under 5% $CO_2$. The test sample was added at 1 mL / well in the experimental group, the action solution of LPS without the sample was added in the model group, and the serum-free 1640 medium without LPS was added in the negative control group. The culture was performed for another 24 hours.

The content of inflammatory factors in the culture supernatant was determined according to the instructions of the ELISA kit for mouse IL-6, TNF-$\alpha$, and IL-1$\beta$, to calculate the relative decrease in inflammatory factors:

$$\text{Relative decrease in inflammatory factors (\%)}= 1 - \frac{\text{the content of inflammatory factors in experimental group}}{\text{the content of inflammatory factors in control group}}$$

As shown in FIG. 4, under LPS stimulation, the pro-inflammatory cytokines IL-6, IL-1$\beta$, and TNF-$\alpha$ secreted by mouse macrophages are significantly reduced, with IL-1 $\beta$ showing the most significant decrease. Taking these three inflammatory factors as the main evaluation indicators, the sample has a certain inhibitory effect on the three inflammatory factors IL-6, TNF - $\alpha$, and IL-1 $\beta$ in mice. The relative values of the three inflammatory factors decrease by 11.5%, 100%, and 8.7% respectively with 1.5% *Cordyceps militaris* fermentation filtrate S1. Therefore, *Cordyceps militaris* fermentation filtrate can effectively reduce skin problems caused by inflammation.

**Test Example** 7: **Melanogenesis Inhibition Test**

1. The effect of the *Cordyceps militaris* fermentation filtrate on the proliferation of B16 mouse melanoma cells

B16 mouse melanoma cells in logarithmic growth phase were taken, inoculated into a 96-well cell culture plate at a density of $2 \times 10^4$ cells/mL, with 100 $\mu$L cell suspension per well, and incubated overnight in a $CO_2$ incubator at 37°C under 5% $CO_2$. The sample S1 of Example 1 was prepared into the concentrations of 1.5% and 3%, respectively using serum containing complete medium, and filtered through a 0.22 $\mu$m filter membrane for sterilization. The old culture solution was discarded, and 1.5%, 3% concentration of sample solution S1 was respectively added in the experimental group, while an equal amount of serum containing cell culture medium was added in the normal control group. The culture was performed for another 24 hours. The relative proliferation rate of the cells was detected by the WST-1 method. The relative proliferation rate (RGR) is the ratio of the absorbance of the experimental group to that of the normal control group.

2. Detection of melanin content

B16 cells in logarithmic growth phase were taken, inoculated into a 6-well culture plate at a density of $2 \times 10^4$ cells/mL, with 3 $\mu$L per well, and incubated for 24 hours in a $CO_2$ incubator at 37°C under 5% $CO_2$. The old culture solution was discarded. 3 mL of serum containing culture medium was added in the normal control group and model group, and 3 mL of sample solution was added in the experimental group. 60 $\mu$L of forskolin solution (2 mM) was added into each well of the model group and experimental group to stimulate production of the melanin. The culture was performed for 72 hours. The sample solutions were 1.5% and 3.0% of *Cordyceps militaris* fermentation filtrate S1.

The method for determining the intracellular melanin content is as follows: digesting with trypsin, centrifuging to remove the supernatant, adding 500L of 1mol/L (containing 10% DMSO) NaOH solution to lyse the cells, heating at 80°C for 30 minutes, centrifuging at 3000rpm for 10 minutes, taking and adding the supernatant to a 96-well plate at 100 $\mu$L/well, measuring the absorbance at 450nm to obtain the total melanin content. The relative value of melanin content in the experimental group was obtained by taking the melanin content in the model group as 100%. If the sample has a significant effect on the proliferation of B16 cells, the ratio of the relative value of melanin content to the corresponding proliferation rate of B16 cells is the melanin production rate of unit cell.

3. Detection of tyrosinase activity

In melanocytes, tyrosine is activated by tyrosinase to form dopa, which is then dehydrogenated to form dopaquinone. The latter rearranges to form 5,6-quinindole, which polymerizes and binds to structural proteins in melanocytes to form melanin. Tyrosinase is a multi-active enzyme in this process, and inhibiting its activity can suppress the formation of melanin. The experimental principle is to determine the relative activity of tyrosinase in the sample by measuring the ability of the tyrosinase contained in the sample to oxidize the substrate dopa to dopaquinone.

B16 cells in logarithmic growth phase were taken, inoculated into a 6-well culture plate at a density of $2 \times 10^4$ cells/mL, with 3 $\mu$L per well, and incubated for 24 hours in a $CO_2$ incubator at 37°C under 5% $CO_2$. The old culture solution was discarded. 3 mL of serum containing culture medium was added in the normal control group and model group, and 3 mL of sample solution was added in the experimental group. 60 $\mu$L of forskolin solution (2 mM) was added into each well of the model group and experimental group to stimulate production of the melanin. The culture was performed for 72 hours. The sample solutions were 1.5% and 3.0% of *Cordyceps militaris* fermentation filtrate S1.

The old culture solution was discarded. The cells were washed twice with PBS, and was lysed with Tirs-HCL (0.02mol/L, pH 6.8) solution containing 0.1% Triton-100, and sonicated for 10 minutes. 0.1% L-dopa (0.01mol/L, pH 8.0) was added. After incubation for 60 minutes at 37°C, centrifugation was performed, and the supernatant was added into a 96-well plate at 100 $\mu$l/well. The absorbance value was measured at 450 nm wavelength using an enzyme-linked immunosorbent assay (ELIASA). The ratio of absorbance between the experimental group and the model group was used as an indicator to evaluate the activity of tyrosinase.

Table 7 Effects of different concentrations of samples on tyrosinase activity and melanin content

| Sample concentration (m/v) | Model group | Experimental group 1.50% | Experimental group 3.00% |
|---|---|---|---|
| Tyrosinase activity (%) | 100.00 | 76.70** | 62.02** |
| Melanin production rate (%) | 100.00 | 81.62** | 63.27** |
| Note: * * indicates that compared to the model group, p<0.001. | | | |

From the results in the table above, it can be seen that different concentrations of *Cordyceps militaris* fermentation filtrate can significantly reduce intracellular tyrosinase activity and melanin production rate. By using *Cordyceps militaris* fermentation filtrate at concentrations of 1.5% and 3.0%, the tyrosinase activity is decreased by 23.3% and 38.0%, respectively, and the melanin production rate is decreased by 18.4% and 36.7%, respectively. Therefore, *Cordyceps militaris* fermentation filtrate can alleviate pigmentation and reduce pigmentation caused by aging.

**Test Example 8: Human Skin Elasticity Test**

8.1. 30 male or female healthy volunteers aged 20-65 years old were recruited. Prior to the test, each subject shall sign an "informed consent form".

8.2. 15 minutes after the subject cleansed the face, initial values of skin elasticity R2 on the left and right sides of the face were detected using an instrument, and recorded.

8.3. The subjects used the samples once a day in the morning and in the evening, in which the mode of use is as follows:

placebo (essence without *Cordyceps militaris* fermentation filtrate) and the test sample (essence containing *1% Cordyceps militaris* fermentation filtrate S1) were used respectively on the left and right sides of the face, and then values of the skin elasticity R2 were tested 1 week, 2 weeks, and 4 weeks after the initial use.

8.4. The relative growth rate of skin elasticity of each subject was calculated based on the following formula, and then the average value is taken.

Relative growth rate of skin elasticity (%)

$$= \frac{(\text{value of skin elasticity } R2 \text{ in the sample group} - \text{value of skin elasticity } R2 \text{ in the placebo group})}{\text{value of skin elasticity } R2 \text{ in the placebo group}}$$

$\times 100\%$

8.5 Test results:

Table 8

| | | Initial | *1 week* | *2 weeks* | *4 weeks* |
|---|---|---|---|---|---|
| Value of skin elasticity R2 (%) | Placebo | 100 | 86 | 79.82 | 72.48 |
| | 1.0%S1 | 100 | 98.59 | 95.75 | 92.68 |
| Relative growth rate of skin elasticity (%) | | 0 | 14.64 | 19.96* | 27.87* |
| Note: * indicates p<0.05 compared with placebo group. | | | | | |

After using the essence containing *1% Cordyceps militaris* fermentation filtrate for 2 weeks, the relative growth rate of skin elasticity of volunteers significantly is increased by 20%. After 4 weeks, the relative growth rate of skin elasticity of volunteers can be increased by 27.87%. Therefore, the *Cordyceps militaris* fermentation filtrate can tighten the skin, and effectively improve the elasticity of aging skin.

**Claims**

1. *A Leuconostoc* sp. HX-236, with a deposit number of CCTCC NO: M20221389.

2. A microbial agent comprising the *Leuconostoc* sp. HX-236 according to claim 1.

3. Use of the *Leuconostoc* sp. HX-236 according to claim 1 or the microbial agent according to claim 2 in the preparation according to a fermentation product; preferably, the fermentation product is a *Cordyceps militaris* fermentation product.

4. A method for preparing a *Cordyceps militaris* fermentation product, comprising a step of fermenting *Cordyceps militaris* with the *Leuconostoc* sp. HX-236 according to claim 1; preferably, the *Cordyceps militaris* is used as a sole nitrogen source for a fermentation medium.

5. A *Cordyceps militaris* fermentation product, wherein the *Cordyceps militaris* fermentation product is obtained by fermenting *Cordyceps militaris* with *Leuconostoc* sp. HX-236 according to claim 1; preferably, the *Cordyceps militaris* is used as a sole nitrogen source for a fermentation medium.

6. The *Cordyceps militaris* fermentation product according to claim 5, wherein the *Cordyceps militaris* fermentation product is a fermentation broth obtained by fermenting *Cordyceps militaris* with *Leuconostoc* sp. HX-236, or made from the fermentation broth as a raw material.

7. The *Cordyceps militaris* fermentation product according to claim 5, wherein a content of cordycepin is 80-200mg/L, and a content of cordycepic acid is 5-30g/L in the *Cordyceps militaris* fermentation product.

8. Use of the *Leuconostoc* sp. HX-236 according to claim 1 or the microbial agent according to claim 2 or the *Cordyceps militaris* fermentation product according to any one of claims 5-7 in the preparation of a medicament and cosmetic.

9. A composition comprising the *Leuconostoc* sp. HX-236 according to claim 1 or the microbial agent according to claim 2 or the *Cordyceps militaris* fermentation product according to any one of claims 5-7.

10. Use of the *Leuconostoc* sp. HX-236 according to claim 1 or the microbial agent according to claim 2 or the *Cordyceps militaris* fermentation product according to any one of claims 5-7 or the composition according to claim 9 in anti-aging, promoting production of collagen I, promoting cell repair and regeneration, anti-inflammatory, whitening, or increasing skin elasticity.

11. A method for anti-aging, promoting the production of collagen I, promoting cell repair and regeneration, anti-inflammatory, whitening, or increasing skin elasticity, wherein the *Leuconostoc* sp. HX-236 according to claim 1, the microbial agent according to claim 2, the *Cordyceps militaris* fermentation product according to any one of claims 5-7, or the composition according to claim 9 is used.

FIG. 1

FIG. 2

FIG. 3

FIG. 4

## INTERNATIONAL SEARCH REPORT

| International application No. |
|---|
| **PCT/CN2023/142667** |

### A.    CLASSIFICATION OF SUBJECT MATTER

C12N1/20(2006.01)i;  C12P1/04(2006.01)i;  A61K35/74(2015.01)i;  A61K8/99(2017.01)i;  A61P29/00(2006.01)i;
A61P17/18(2006.01)i;  A61P17/16(2006.01)i;  A61Q19/02(2006.01)i;  A61Q19/08(2006.01)i;  C12R1/01(2006.01)n

According to International Patent Classification (IPC) or to both national classification and IPC

### B.    FIELDS SEARCHED

Minimum documentation searched (classification system followed by classification symbols)

IPC:C12N,C12P,A61K,A61P,A61Q,C12R

Documentation searched other than minimum documentation to the extent that such documents are included in the fields searched

Electronic data base consulted during the international search (name of data base and, where practicable, search terms used)

DWPI, CNTXT, ENTXTC, ENTXT, CNKI, 万方, WANFANG, ISI Web of Science, Pubmed, Elsevier Science, 中国专利生物序列检索系统, China Patents Biological Sequence Search System, Genbank, EMBL, Ensembl: 明串珠菌, 明串菌, 乳酸菌, 蛹虫草, 北冬虫夏草, 北虫草, 蛹草, 发酵液, 虫草素, 虫草酸, 胶原蛋白, 抗炎, 美白, Leuconostoc sp., lactic acid bacteria, Cordycepsmilitaris, Cordyceps militaris, North cordyceps sinensis, fermentation extract liquid, cordycepin, cordycepic acid, collogen, antiinflammatory, whitening, skin-white, 针对SEQ ID NO: 1进行的序列检索, sequence search for SEQ ID NO: 1

### C.    DOCUMENTS CONSIDERED TO BE RELEVANT

| Category* | Citation of document, with indication, where appropriate, of the relevant passages | Relevant to claim No. |
|---|---|---|
| X | CN 111808901 A (BLOOMAGE BIOTECHNOLOGY CORPORATION LIMITED; SHANDONG BLOOMAGE HYINC BIOPHARM CO., LTD.) 23 October 2020 (2020-10-23) see abstract, and description, paragraphs 14, 19-21 and 57-71 | 1-11 |
| A | CN 110651936 A (TIANJIN LIANGXIN INSPECTION AND CERTIFICATION TECHNOLOGY CO., LTD.; TIANJIN INDUSTRIAL MICROBIOLOGY RESEARCH INSTITUTE CO., LTD.; TIANJIN SF-BIO INDUSTRIAL BIO-TEC CO., LTD.) 07 January 2020 (2020-01-07) see abstract | 1-11 |
| A | CN 113317423 A (SHANDONG XIAOFENG BIOTECHNOLOGY CO., LTD.) 31 August 2021 (2021-08-31) see abstract | 1-11 |

| ✓ Further documents are listed in the continuation of Box C. | ✓ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **23 February 2024** | **23 March 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2023/142667** |

**C.  DOCUMENTS CONSIDERED TO BE RELEVANT**

| Category* | Citation of document, with indication, where appropriate, of the relevant passages | Relevant to claim No. |
|---|---|---|
| A | KWON, H. K. et al. "Immune-enhancing activity of C. militaris fermented with Pediococcus pentosaceus (GRC-ON89A) in CY-induced immunosuppressed model" *BMC Complement Altern Med.*, Vol. 18, No. 1, 23 February 2018 (2018-02-23), see abstract | 1-11 |

Form PCT/ISA/210 (second sheet) (July 2022)

## INTERNATIONAL SEARCH REPORT

| International application No. |
| --- |
| **PCT/CN2023/142667** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

<table>
<tr><td colspan="2">International application No.</td></tr>
<tr><td colspan="2">**PCT/CN2023/142667**</td></tr>
</table>

| Patent document cited in search report | | | Publication date (day/month/year) | Patent family member(s) | Publication date (day/month/year) |
|---|---|---|---|---|---|
| CN | 111808901 | A | 23 October 2020 | None | |
| CN | 110651936 | A | 07 January 2020 | None | |
| CN | 113317423 | A | 31 August 2021 | None | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 111808901 A